# EUROPÄISCHE PATENTSCHRIFT

(11) **EP 0 538 550 B1**
(45) Veröffentlichungstag und Bekanntmachung des Hinweises auf die Patenterteilung: **11.12.1996**
(21) Anmeldenummer: 92108646.8
(22) Anmeldetag: 22.05.1992
(51) Int. Cl.: A47B 88/04, B67D 1/04

(54) **Einrichtung**
Device
Dispositif

(30) Priorität: 24.10.1991 DE 9113223 U; 30.01.1992 DE 9201086 U
(43) Veröffentlichungstag der Anmeldung: 28.04.1993
(73) Patentinhaber: KARL BAISCH GmbH, D-71384 Weinstadt (DE)
(72) Erfinder: Grunert, Hans Christof, D-73099 Adelberg (DE)
(74) Vertreter: Kohl, Karl-Heinz

(56) Entgegenhaltungen:
- EP-A- 0 106 972
- CA-A- 1 267 661
- FR-A- 1 411 223
- FR-A- 2 336 101
- FR-A- 2 557 554
- GB-A- 1 055 552
- US-A- 2 742 048
- US-A- 3 880 345
- US-A- 4 596 427
- US-A- 4 792 059

## Beschreibung

Die Erfindung betrifft eine Einrichtung nach dem Oberbegriff des Anspruches 1.

Bei bekannten, als Möbel ausgebildeten Einrichtungen ist das bewegbare Teil eine Schublade, die an ihrer Blende einen Griff trägt, mit dem die Schublade zum Öffnen und Schließen erfaßt werden kann. Insbesondere bei Instrumenten- oder Medikamentenschränken, die in Arzt- oder Zahnarztpraxen aufgestellt sind, kommt es immer wieder vor, daß der Arzt bzw. Zahnarzt während der Behandlung die Schublade öffnen muß, um ein neues oder ein anderes Instrument zu entnehmen. Hierbei muß er die Schublade am Griff herausziehen und anschließend wieder hineinschieben. Dies ist umständlich und zeitaufwendig, insbesondere dann, wenn der Arzt oder Zahnarzt seine eine Hand nicht frei hat, sondern beispielsweise einen Zahnbohrer oder dergleichen noch in der Hand hält.

Bei Arzt- und Zahnarztpraxen ist es auch bekannt, an der Wand Spender für Seife und/oder Desinfektionsmittel zu befestigen. Der Spender hat einen Schwenkarm als bewegbares Teil, der in der Regel mit dem Ellenbogen oder dem Unterarm betätigt wird, um das entsprechende Mittel aus dem Behältnis herauszupumpen. Eine derartige Betätigung des Spenders ist umständlich, insbesondere wenn der Benutzer seine Hände nicht frei hat.

Bei der gattungsgemäßen Einrichtung (US-A-3 880 345) wird eine Schublade mit einer Antriebseinrichtung zwischen zwei verschiedenen Stellungen hin- und hergeschoben. Die Antriebseinrichtung ist eine pneumatische Kolben-Zylinder-Einheit, deren Kolbenstange an einen Lenker angelenkt ist, der seinerseits gelenkig mit der Schublade verbunden ist. Zur Betätigung der Antriebseinrichtung ist ein Drehschalter vorgesehen, der zwischen zwei verschiedenen Stellungen verstellt werden kann. Die Bedienungsperson muß darum wenigstens eine Hand frei haben, um den Drehschalter betätigen zu können. Darum ist diese Einrichtung dort nicht geeignet, wo die Bedienungsperson zur Betätigung des Drehschalters die Hand nicht frei hat.

Es ist auch bekannt (EP-A2-0 106 972), aus einem Vorratsbehälter über ein Einlaßventil Frischmilch in ein Meßgefäß zu fördern. Ist es gefüllt, strömt die Milch über eine Leitung und ein druckluftgesteuertes Auslaßventil in eine Verbraucherflasche.

Es ist ferner bekannt (FR-A-1 411 223), die Schublade eines Möbels mit einer Kolben-Zylinder-Einheit zu verschieben. Der Kolben der Einheit ist beidseitig mit Druckluft beaufschlagbar, wofür eine Antriebsvorrichtung vorgesehen ist. Sie hat einen federbelasteten Schieber, der von Hand so verstellt werden muß, daß der entsprechende Druckraum mit Druckmedium beaufschlagt wird. Da der Schieber unter Federkraft steht, muß er so lange gezogen bzw. gedrückt gehalten werden, bis die Schublade in die gewünschte Stellung verschoben worden ist.

Bei Fenstern ist es bekannt (CA-A-1 267 661), über ein Kabel ein zu verschiebendes Fenster mit einer Kolbenstange einer pneumatischen Antriebseinrichtung zu verbinden. Durch Ein- und Ausfahren der Kolbenstange wird das Fenster über das Kabel entsprechend vertikal verschoben.

Bei einer Ausschankeinrichtung für Bier ist es bekannt (FR-A1-2 557 554), Bier aus einem Vorratsbehälter mittels Druckluft zu fördern. Die Ausschankeinrichtung hat Schaltventile, die zum Fördern des Bieres sowie entsprechender Zusätze verstellt werden.

Es ist schließlich ein Getränkeautomat bekannt (US-A-4 792 059), der Schalter aufweist, mit denen die Zuführung von Flüssigkeit zu einem Auslaß gesteuert werden kann. Mit den Schaltern wird jeweils eine Klemmstange verstellt, mit der aus elastischem Werkstoff bestehende Flüssigkeitsleitungen zusammengequetscht oder geöffnet werden. Um Flüssigkeit dem Getränkeautomaten zu entnehmen, muß der entsprechende Schalter jeweils so lange von Hand gedrückt gehalten werden, bis die entsprechende Flüssigkeitsmenge aus dem Getränkeautomaten geflossen ist.

Der Erfindung liegt die Aufgabe zugrunde, die gattungsgemäße Einrichtung so auszubilden, daß das zu betätigende Teil mit der Antriebsvorrichtung auch dann betätigt werden kann, wenn die Bedienungsperson keine Hand frei hat.

Diese Aufgabe wird bei der gattungsgemäßen Einrichtung erfindungsgemäß mit den kennzeichnenden Merkmalen des Anspruches 1 gelöst.

Bei der erfindungsgemäßen Einrichtung kann der Auslöser mit dem Fuß, dem Bein oder mit dem Arm der Bedienungsperson einfach gedrückt werden. Der Auslöser erzeugt einen ersten Schaltimpuls, durch welchen die Antriebsvorrichtung betätigt und dadurch das zu betätigende Teil, beispielsweise eine Schublade eines Instrumentenschrankes, ausgefahren wird. Zur Betätigung des Auslösers ist die Hand der Bedienungsperson nicht notwendig. Dies ist besonders für Ärzte und Zahnärzte aus hygienischen Gründen sehr vorteilhaft, da sie zum Öffnen und Schließen mit ihren Händen das zu betätigende Teil, wie eine Schublade, einen Spender oder dergleichen, nicht erfassen und während der Behandlung zum Beispiel ihre Instrumente nicht weglegen müssen. Auch behinderte Personen, wie zum Beispiel contergangeschädigte Personen, armamputierte Personen und dergleichen können den Auslöser ohne Schwierigkeiten betätigen. Soll das zu betätigende Teil wieder in die Ausgangslage zurückgeführt werden, ist es lediglich notwendig, den Auslöser erneut zu drücken. Hierbei wird ein zweiter Schaltimpuls erzeugt, durch den die Antriebsvorrichtung entsprechend betätigt wird. Die erfindungsgemäße Einrichtung ist somit hervorragend im Arzt-/Zahnarztbereich einsetzbar, um beispielsweise Schubladen von Instrumentenschränken zu öffnen bzw. zu schließen. Während des Schließvorganges kann die Bedienungsperson ihre Arbeit bereits wieder aufnehmen.

Weitere Merkmale der Erfindung ergeben sich aus den abhängigen Ansprüchen 2 bis 32.

Die Erfindung wird nachstehend anhand mehrerer in den Zeichnungen dargestellter Ausführungsbeispiele näher beschrieben. Es zeigt:
- Figur 1: in Draufsicht eine erfindungsgemäße, als Möbel ausgebildete Einrichtung mit einem Möbelkorpus und einer Schublade, die in Schließ- und in Offenstellung dargestellt ist,
- Figur 2: eine zweite Ausführungsform einer erfindungsgemäßen, als Möbel ausgebildeten Einrichtung mit einem Möbelkorpus, in dem mehrere übereinander angeordnete Schubladen angeordnet sind,
- Figur 3: eine weitere Ausführungsform einer erfindungsgemäßen, als Möbel ausgebildeten Einrichtung in einer Darstellung entsprechend Figur 1,
- Figur 4: eine vierte Ausführungsform einer als Möbel ausgebildeten Einrichtung in einer Darstellung entsprechend Figur 1,
- Figur 5: in schematischer Darstellung und in Vorderansicht ein weiteres Ausführungsbeispiel einer erfindungsgemäßen Einrichtung mit einem integrierten Handwaschbecken,
- Figur 6: die Einrichtung mit Handwaschbecken nach Figur 5 in Seitenansicht gemäß Pfeil VI in Figur 5.

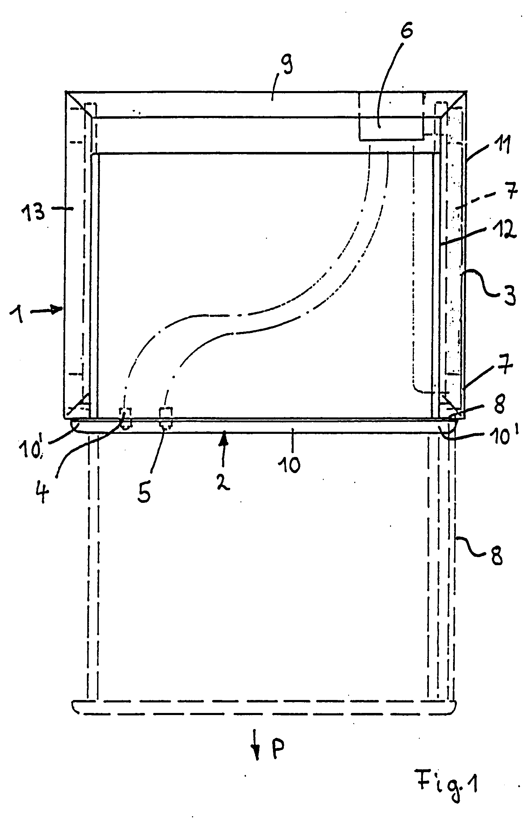

Bei den Ausführungsformen nach den Figuren 1 bis 4 ist die Einrichtung jeweils als Möbel ausgebildet. Das Möbel nach Figur 1 kann beispielsweise ein Schrank in einer Apotheke, in einem Arzt- oder Zahnarztzimmer oder in einer Bank, ein Küchenschrank, ein Schrank in einem Juweliergeschäft oder auch eine Theke in einer Tankstelle oder dergleichen sein. Das Möbel hat einen Möbelkorpus 1 mit Schubladen 2, von denen wenigstens eine, vorzugsweise die oberste Schublade, zwischen einer Schließ- und einer Offenstellung verschiebbar ist. Die Offenstellung ist mit gestrichelten Linien dargestellt. Zum Betätigen der Antriebsvorrichtung 3 sind ein elektrischer Ein- und Ausschalter 4 und 5 vorgesehen, die mit einem Schaltventil 6 der Antriebsvorrichtung 3 verbunden sind. In der ausgefahrenen Endstellung (gestrichelte Linien in Figur 1) ist die Kolbenstange 8 ebenfalls aus dem Zylinder 7 ausgefahren. Die Antriebsvorrichtung ist eine pneumatische Kolben-Zylinder-Einheit, die in Ausziehrichtung P der Schublade 2 verläuft. Sie ist zwischen der einen Seitenwand 11 und der zu ihr parallelen Seitenwand 12 der Schublade 2 angeordnet. Der Zylinder 7 der Antriebsvorrichtung kann an der Rückwand 9 oder an der Seitenwand 11 des Korpus 1 befestigt sein, während die Kolbenstange 8 an einem seitlich über die Seitenwand 12 der Schublade 2 überstehenden Abschnitt 10' einer Blende 10 der Schublade 2 angreift. Durch die Blende 10 ist die Antriebsvorrichtung 3 nach vorn abgedeckt. Die Kolbenstange 8 kann auch an einem mit der Seitenwand 12 der Schublade 2 fest verbundenen Anschlußteil angreifen.

Bei einem Möbel mit mehreren Schubladen kann jeder Schublade jeweils eine Kolben-Zylinder-Einheit 3 zugeordnet sein. Auf diese Weise ist es möglich, beispielsweise die oberen zwei, drei oder auch mehrere Schubladen durch Beaufschlagung des jeweiligen Pneumatikzylinders ein- und auszufahren.

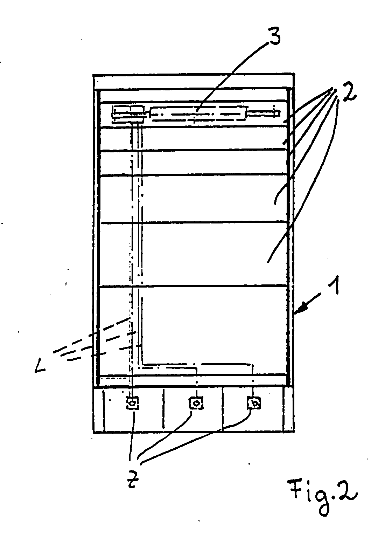

Figur 2 zeigt ein Möbel, das mehrere übereinander angeordnete Schubladen 2 hat, deren Höhe von oben nach unten zunimmt. An der oberen und den beiden darunterliegenden Schubladen greift jeweils eine Kolben-Zylinder-Einheit 3 an, von denen - der Übersichtlichkeit wegen - nur die obere Kolben-Zylinder-Einheit dargestellt ist. Die Kolben-Zylinder-Einheiten 3 werden über Schalteinrichtungen Z betätigt, die im Fußbereich des Möbels 1 angeordnet sind.

Von den Einheiten 3 bzw. einem ihnen gemeinsamen Schaltventil führen Leitungen L zu den Schalteinrichtungen Z. Anstelle eines gemeinsamen Schaltventils für alle Kolben-Zylinder-Einheiten 3 kann jeder Einheit auch ein eigenes Schaltventil zugeordnet sein.

Zum Auslösen der Antriebsvorrichtungen 3 sind die im Fußbereich des Möbels 1 angeordneten Schalteinrichtungen Z vorgesehen. Sie können so ausgebildet sein, daß pro Pneumatikzylinder ein Einschalter und für alle Pneumatikzylinder ein Ausschalter vorhanden ist. Mit den Einschaltern wird die entsprechende Schublade ausgewählt und ausgefahren. Mit dem Ausschalter werden sämtliche ausgefahrenen Kolbenstangen zum Schließen der entsprechenden Schubladen wieder zurückgefahren.

Bei einer anderen Ausführungsform wird für jeden Pneumatikzylinder nur ein Schalter benötigt, der so ausgebildet ist, daß durch das erste Betätigen die jeweilige Kolbenstange 1 ausgefahren und durch ein erneutes Betätigen zurückgefahren wird.

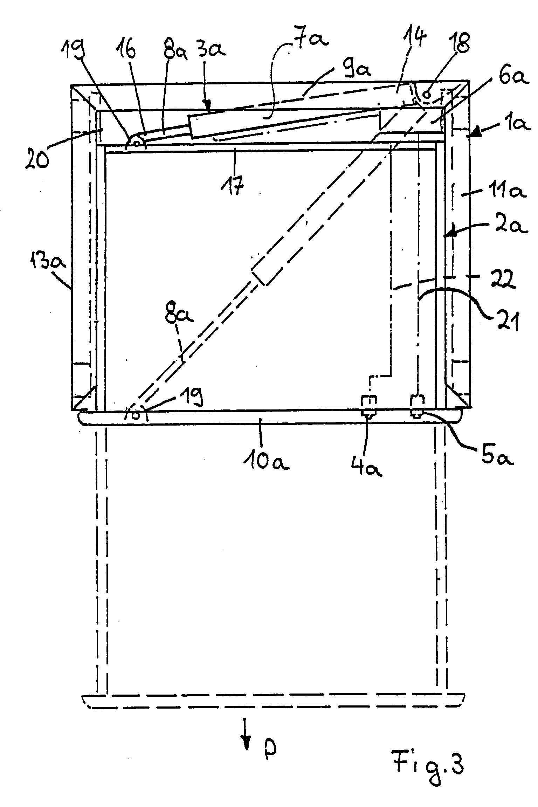

Die Ausführungsform nach Figur 3 unterscheidet sich von der zuvor beschriebenen Ausführungsform im wesentlichen dadurch, daß die pneumatische Kolben-Zylinder-Einheit nicht seitlich neben, sondern hinter der Schublade 2a und schräg zu ihrer Ausfahrrichtung P liegt.

Das freie Ende 14 des Zylinders 7a der Kolben-Zylinder-Einheit 3a ist in einem Eckbereich 15 an der Möbelrückwand 9a gelenkig befestigt (Anlenkstelle 18), während das freie Ende 16 der Kolbenstange 8a an einer Rückwand 17 der Schublade 2a angelenkt ist (Anlenkstelle 19). Die Anlenkstellen 18 und 19 liegen jeweils benachbart zu der einen bzw. anderen Seitenwand 11a bzw. 13a des Möbelkorpus 1a, wobei die Anlenkstelle 18 vorzugsweise unmittelbar benachbart zur Seitenwand 11a liegt. In jedem Fall liegt die Anlenkstelle 19 zumindest geringfügig neben der in Ausfahrrichtung P sich erstreckenden Mittelebene der Schublade 2a.

Diese Anordnung der Antriebsvorrichtung 3a hat den Vorteil, daß auf die Schublade beim Ein- und Ausfahren nur verhältnismäßig geringe Querkräfte wirken. Dadurch sind eine exakte Führung und damit ein geringer Verschleiß der Schublade 2a sichergestellt und eine Beschädigung der Schublade und der Schubladenführung vermieden. Außerdem läßt sich die Schublade leichtgängig öffnen und schließen.

Bei der ausgefahrenen, in Figur 3 mit gestrichelten Linien dargestellten Lage ist die Kolbenstange 8a aus dem Zylinder 7a ausgefahren und liegt, wie auch der Zylinder, nahezu diagonal zur Aufnahmeöffnung des Möbelkorpus 1a für die Schublade 2a. Zum Betätigen der Antriebsvorrichtung 3a sind wie bei der Ausführungsform nach Figur 1 ein Ein- und Ausschalter 4a und 5a vorgesehen, die über elektrische Leitungen 21, 22 mit dem Schaltventil 6a verbunden sind, mit dem der Pneumatikzylinder 7a gesteuert wird. Die Schalter 4a, 5a liegen wie die Schalter 4 und 5 im unteren Bereich des Möbelkorpus 1a.

Bei eingefahrener Schublade 2a (ausgezogene Linien in Figur 3) erstreckt sich der Pneumatikzylinder 7a von seiner Anlenkstelle 18 an der Möbelrückwand 9a aus in Ausfahrrichtung P der Schublade 2a schräg nach vorn. Dadurch ist sichergestellt, daß beim Ausfahren der Kolbenstange 8a die Schublade 2a ohne größeren Kraftaufwand aus dem Möbelkorpus 1a geschoben werden kann. Da der Pneumatikzylinder 7a durch die Schublade verdeckt wird, ist er von vorn nicht sichtbar. Je nach Ausziehlänge der Schublade 2a kann die Anlenkung 19 der Kolbenstange 8a an der Rückwand 17 der Schublade unterschiedlichen Abstand von der in Ausfahrrichtung P sich erstreckenden Mittelebene der Schublade haben. Je näher die Anlenkstelle 19 an dieser Mittelebene liegt, desto geringer ist die Querbelastung der Schublade 2a beim Ausfahren mit dem Pneumatikzylinder 7a.

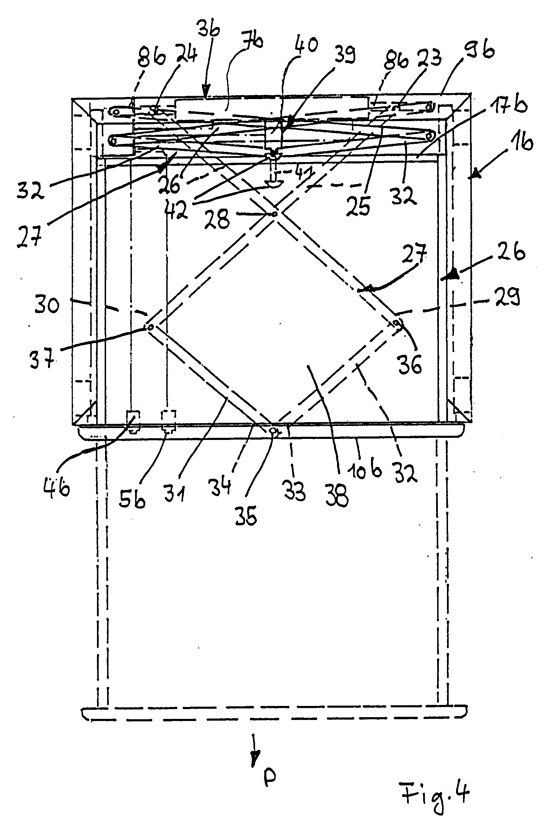

Bei der Ausführungsform nach Figur 4 ist die Antriebsvorrichtung 3b zwischen der Rückwand 9b des Möbelkorpus 1b und der Rückwand 17b der Schublade 2b vorgesehen. Der Zylinder 7b liegt vorzugsweise parallel zur Rückwand 9b. Über beide Enden des Zylinders 9b ragt jeweils eine Kolbenstange 8b, 8b', an deren freien Enden jeweils ein Ende 23, 24 eines Armes 25 und 26 einer scherenartigen Kraftübertragungsvorrichtung 27 angreift. Die vorzugsweise gleich langen Arme 25 und 26 sind an den Kolbenstangen 8b und 8b' angelenkt und in halber Länge gelenkig miteinander verbunden (Gelenkstelle 28). An den von den Kolbenstangen 8b, 8b' abgewandten Enden 29 und 30 der Arme 24 und 25 ist jeweils ein Ende von weiteren Armen 31 und 32 angelenkt (Gelenkstellen 36 und 37), die an ihren anderen Enden 33 und 34 über eine Gelenkstelle 35 miteinander und mit der Blende 10b der Schublade 2b verbunden sind. Vorzugsweise sind die Arme 31 und 32 etwa halb so lang wie die Arme 24 und 25. Zwischen den Gelenkstellen 28, 35 und 36, 37 ist ein parallelogrammförmiges Gelenkviereck 38 gebildet. Der Pneumatikzylinder 7b ist so ausgebildet, daß seine beiden Kolbenstangen 8b, 8b' bei Druckbeaufschlagung synchron aus dem Zylinder ausgefahren und bei entsprechender Umschaltung synchron eingefahren werden.

Beim Betätigen der Antriebsvorrichtung 3b werden die Kolbenstangen 8b und 8b' somit in entgegengesetzte Richtungen zueinander ausgefahren, wobei sich die Kraftübertragungsvorrichtung 27 aus ihrer mit ausgezogenen Linien dargestellten zusammengelegten Lage in ihre gestreckte Lage (gestrichelte Linien) bewegt. Dabei werden die Arme 23 und 24 scherenartig gegeneinander geschwenkt, wobei die Arme 31 und 32 zusammen mit der Schublade 2b, an der sie bei der Anlenkstelle 35 angreifen, nach außen in Richtung des Pfeiles P bewegt werden.

Um das Ausfahren der Schublade 2b zu erleichtern, ist bei der Ausführungsform nach Figur 4 eine zusätzliche Kolben-Zylinder-Vorrichtung 39 vorgesehen, mit der bei Beginn des Ausfahrens der Kolbenstangen 8b und 8b' ein toter Weg der Kraftübertragungsvorrichtung 27 überwunden werden kann. Ist die Schublade 2b eingeschoben, dann sind die Arme der Kraftübertragungsvorrichtung 27 zusammengefahren. Ohne den Zusatzantrieb in Form der Kolben-Zylinder-Vorrichtung 39 müßte der Pneumatikzylinder 7b verhältnismäßig hohe Kräfte ausüben, um die zusammengelegten Arme der Kraftübertragungsvorrichtung 27 zu öffnen. Durch den Zusatzantrieb 39 ist sichergestellt, daß die Arme 25, 26, 31, 32 der Kraftübertragungsvorrichtung 27 zu Beginn des Ausfahrens der Schublade 2b durch den Zusatzantrieb 39 etwas auseinandergeschwenkt werden, so daß die Arme 25 und 26 unter einem größeren Winkel zu den Kolbenstangen 8b, 8b' liegen. Sie können dann mit geringer Kraft die Arme 25, 26 in die mit gestrichelten Linien dargestellte Lage schwenken. Somit ist sichergestellt, daß die Schublade 2b gleichmäßig, das heißt mit nahezu konstanter Geschwindigkeit und druckfrei ausgefahren werden kann.

Der Zusatzantrieb 39 hat einen Zylinder 40 und eine Kolbenstange 41, die sich in Ausfahrrichtung P der Schublade 2b erstrecken. Der Zylinder 40 ist in halber Länge am Pneumatikzylinder 7b befestigt. Das freie Ende der Kolbenstange 41 trägt als Anschlag einen Gummipuffer 42 oder dergleichen, mit dem die Vorrichtung 39 beim Ausfahren ihrer Kolbenstange 41 an die Rückwand 17b der Schublade 2b anstößt und dadurch die Schublade bei Druckbeaufschlagung der Antriebsvorrichtung 3b nach vorne schiebt. Dadurch wird im Moment des Auftreffens des Puffers 42 auf die Schublade 2b die Kraftübertragungsvorrichtung 27 in der beschriebenen Weise geöffnet, so daß die Vorrichtung 27 entlastet wird. Der quer zur Ausfahrrichtung P liegende Zylinder 7b kann dann mit seinen Kolbenstangen 8b, 8b' die scherenartige Kraftübertragungsvorrichtung 27 mit verhältnismäßig geringer Kraft öffnen, das heißt ihre Arme 25, 26 und 31, 32 in Richtung P strecken, wobei die Schublade 2b nach vorn bewegt wird. Die Kolben-Zylinder-Einheit 3b wird wie bei der Ausführungsform nach Figur 1 mit einem Ein- und Ausschalter 4b und 5b ein- bzw. ausgeschaltet.

Die beiden Pneumatikzylinder 7b und 40 haben einen gemeinsamen Zylinderraum, so daß die Kolbenstangen 8b, 8b' und 41 gleichzeitig beaufschlagt werden. Da die Kolbenstange 41 des Zusatzantriebes 39 nicht mit der Schublade 2b verbunden ist, kann sie bei Druckbeaufschlagung schneller ausfahren als die Kolbenstange 8b, 8b', an denen die Arme 25 und 27 der Kraftübertragungsvorrichtung 27 angelenkt sind. Dadurch wird die Schublade 2b zunächst durch die ausfahrende Kolbenstange 41 in Ausfahrrichtung P bewegt, wodurch die scherenartige Kraftübertragungsvorrichtung 27 teilweise geöffnet wird und die Kolbenstangen 8b, 8b' anschließend mit geringer Kraft die Kraftübertragungsvorrichtung 27 zum vollständigen Ausfahren der Schublade 2b öffnen können. Die Rückwand 17b der Schublade 2b hebt hierbei von der Kolbenstange 41 des Zusatzantriebes 39 ab.

Da die Antriebsvorrichtung 3b, die Kraftübertragungsvorrichtung 27 und der Zusatzantrieb 39 symmetrisch zu der in Ausfahrrichtung P der Schublade 2b sich erstreckenden Mittelebene liegen, wird die Schublade beim Ein- und Ausfahren nicht durch Querkräfte belastet, so daß sie in ihren Führungen nicht verkanten kann und gleichmäßig verfahren wird.

Werden die Kolbenstangen 8b, 8b' wieder eingefahren, dann wird die Kraftübertragungsvorrichtung 27 geschlossen und somit die Schublade 2b zurückgefahren. Gegen Ende ihres Einzugweges läuft die Rückwand 17b auf die ausgefahrene Kolbenstange 41 des Zusatzantriebes 39 auf, die dann beim weiteren Einfahren der Schublade 2b in den Zylinder 40 zurückgeschoben wird. Wie bei den vorigen Ausführungsformen läuft die Blende 10b der Schublade 2b gegen die Seitenwände des Möbelkorpus 1b auf, wodurch die Einfahrbewegung der Schublade beendet ist und das (nicht dargestellte) Schaltventil ein entsprechendes Signal zum Umschalten erhält, so daß die Pneumatikzylinder 7b und 40 nicht mehr beaufschlagt werden.

Die Antriebsvorrichtung 3b, die Kraftübertragungsvorrichtung 27 und der Zusatzantrieb 39 liegen wie beim vorigen Ausführungsbeispiel geschützt im Bereich zwischen der Rückwand 17b der Schublade 2b und der Rückwand 9b des Möbelkorpus 1b. Infolge ihrer scherenartigen Ausbildung nimmt die Kraftübertragungsvorrichtung 27 in ihrer zusammengelegten Lage nur wenig Platz in Anspruch, so daß die Schublade 2b in der eingefahrenen Stellung bis nahe an die Rückwand 9b des Möbelkorpus 1b reichen kann.

Bei den beschriebenen pneumatischen Antriebsvorrichtungen 3, 3a, 3b läßt sich die Geschwindigkeit, mit der die einzelnen Schubladen ausgefahren werden, einstellen. Hierzu ist ein (nicht dargestelltes) Einstellelement, vorzugsweise eine Drossel, am Schaltventil vorgesehen, mit der die Durchflußgeschwindigkeit der Druckluft und damit die Ausfahrgeschwindigkeit der Kolbenstangen eingestellt werden kann. Mit diesen Drosseln läßt sich vom Benutzer somit die Ausfahrgeschwindigkeit der Schubladen sehr einfach und feinfühlig einstellen. Die Drosseln können jederzeit auch nachträglich eingebracht werden.

Anstelle der beschriebenen pneumatischen Antriebe 3, 3a, 3b kann auch eine (nicht dargestellte) elektrische Antriebsvorrichtung vorgesehen sein. Als Antriebselement dient in diesem Fall vorteilhaft ein Reibrad, dessen Drehachse senkrecht zur Ausfahrrichtung P der Schublade liegt und im Bereich unterhalb der jeweils auszuziehenden Schublade angeordnet ist. Die Schublade weist an ihrer Unterseite bzw. ihrem Boden und durch die vordere Blende verdeckt angeordnet eine Leiste oder dergleichen auf, an der das Reibrad reibschlüssig anliegt. Je nach Drehrichtung des Elektromotors und damit des Reibrades wird die Schublade ein- oder ausgefahren.

Der elektromotorische Antrieb ist vorteilhaft so ausgebildet, daß er ausschließlich elektrisch betätigt werden kann. Wird versucht, die Schublade von Hand zu öffnen, dann wirkt auf das Reibrad eine wesentlich größere Kraft, als sie vom Elektromotor an sich ausgeübt wird. Diese übermäßige Kraft führt dazu, daß eine Warneinrichtung betätigt werden kann, so daß ein gewisser Diebstahlschutz gegeben sein kann.

Die beschriebenenen Ausführungsbeispiele werden bevorzugt für Arzt- und Zahnarztschränke mit Schubladen eingesetzt. Weitere Anwendungsmöglichkeiten sind Schubladen beispielsweise im Hotel- und Küchenbereich, bei Juwelieren, bei Banktresen und dergleichen. Gerade bei Verwendung der pneumatisch angetriebenen Schubladen im Juwelier- und Bankbereich tritt der Vorteil auf, daß solche Schubladen keine Griffe mehr benötigen. Dadurch ist ein gewisser Sicherheitsfaktor gegeben, weil diese Schubladen von Hand nur gegen die Kraft der Pneumatikzylinder geöffnet werden können. Dies läßt sich nicht schnell und einfach bewerkstelligen, so daß ein gewisser Diebstahlschutz gegeben ist. Solche Schubladen können auch in Läden eingesetzt werden, insbesondere in solchen Läden, die höherwertige Waren anbieten. Hier wirkt sich der beschriebene Diebstahlschutz besonders vorteilhaft aus.

Ein weiteres Einsatzgebiet solcher pneumatischer oder motorischer Antriebe für Schubladen ist zum Beispiel der Tankstellenbereich. Dort tritt in der Regel das Problem auf, daß die Bedienungsperson nicht ständig hinter der Theke stehen kann, so daß durch den beschriebenen Diebstahlschutz verhindert werden kann, daß die Schubladen der Theken unbefugt geöffnet werden können.

Ein weiterer wesentlicher Vorteil der beschriebenen Lösungen besteht darin, daß die Schubladen, Schranktüren und dergleichen insbesondere von Behinderten bedient werden können. Dies gilt dann besonders, wenn die entsprechenden Auslöser bzw. Schalter für die Antriebsvorrichtung im Fußbereich angeordnet sind. Behinderte Personen können dann ohne Schwierigkeiten die entsprechenden Schubladen, Türen usw. öffnen und wieder schließen. Die Anordnung der Schaltvorrichtungen im Fußbereich ist besonders in Arzt- und Zahnarztpraxen sehr vorteilhaft, wo hohe Anforderungen an die Hygiene gestellt werden. Bei einer Fußbetätigung kann der Arzt während der Behandlung mit dem Fuß die Schublade aus- und einfahren, ohne daß er hierzu an die Schublade fassen muß. Dadurch entfällt das umständliche Sterilisieren der Hände beim Berühren der Schubladen.

Die Auslöser müssen nicht zwingend im Fußbereich angeordnet sein; sie können auch als Schaltleisten beispielsweise in Kniehöhe, in Höhe der Hände usw. liegen. Die zuletzt genannte Möglichkeit ist besonders für Contergangeschädigte vorteilhaft, die dadurch die Bedienungselemente mit den Händen erfassen können.

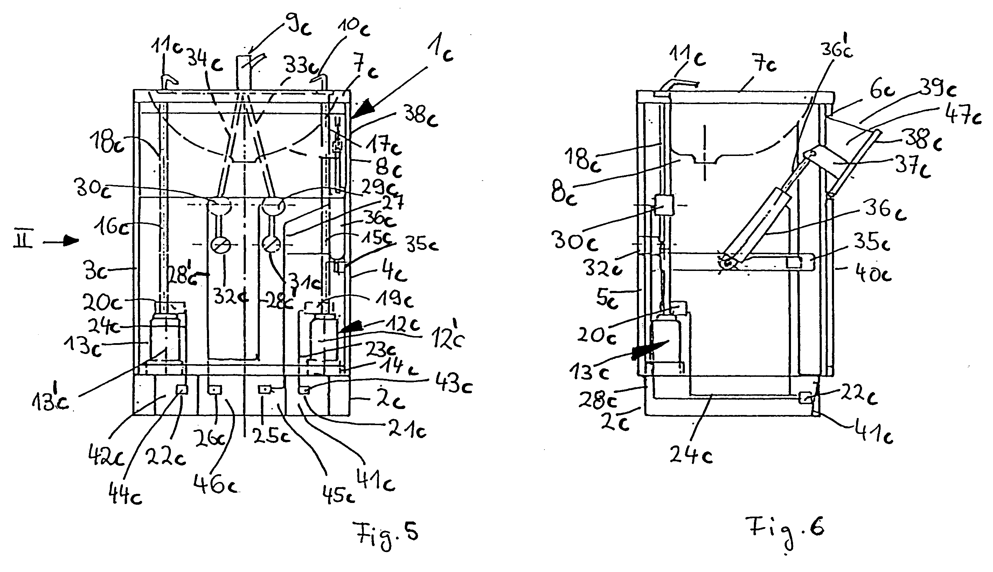

Die Einrichtung 1c nach den Figuren 5 und 6 ist vorteilhaft als Schrank ausgebildet und hat einen Sockel 2c, Seitenwände 3c und 4c sowie eine Rück- und Vorderwand 5c und 6c, auf denen eine Deckplatte 7c ruht. In der Deckplatte 7c sind eine zentrale (nicht dargestellte) Öffnung für ein Handwaschbecken 8c und (ebenfalls nicht dargestellte) Durchtrittsöffnungen für einen Wasserhahn 9c und zwei Auslaufstutzen bzw. -düsen 10c und 11c vorgesehen, von denen der eine Teil eines Seifenspenders 12c und der andere Teil eines Spenders 13c für ein Desinfektionsmittel ist.

Die Spender 12c und 13c haben jeweils einen Behälter 12'c und 13'c, der im Schrank 1c vorzugsweise benachbart zu den Seitenwänden 3c und 4c platzsparend auf einem Boden 14c angeordnet ist, der auf dem Sockel 12c liegt.

Die Behälter 12'c, 13'c sind über jeweils eine in Figur 5 mit strichpunktierten Linien angedeutete Steigleitung 15c, 16c mit dem Auslaufstutzen 10c, 11c verbunden. Die Steigleitungen 15c, 16c liegen innerhalb jeweils eines Einfüllrohres bzw. -schlauches 17c, 18c, die in eine zugehörige Öffnung der Platte 7c münden bzw. an den Öffnungsrand angeschlossen sind. Zum Befüllen der Behälter 12'a, 13'a können die entsprechenden Flüssigkeiten von der Oberseite des Schrankes 1c aus eingefüllt werden, indem die Flüssigkeit in den zwischen dem Steigrohr 15c, 16c und der Innenwand des das Steigrohr mit Abstand umgebenden Einfüllschlauches 17c, 18c gebildeten Ringkanal gefüllt wird. Dadurch lassen sich die Behälter 12'c, 13'c von der Oberseite des Schrankes aus bequem nach- oder auffüllen. Die Spender 12c, 13c haben jeweils eine Pumpvorrichtung 19c, 20c, die im Bereich oberhalb der Spenderbehälter 12'c, 13'c vorgesehen ist. Diese Pumpvorrichtungen sind jeweils über einen luftgesteuerten Schalter 21c, 22c betätigbar, der vorzugsweise ein Mikroschalter ist. Er ist über eine Druckluftleitung 23c, 24c mit der jeweiligen Pumpvorrichtung 19c, 20c verbunden.

Die Mikroschalter 21c, 22c sind an der Vorderseite des Schrankes 1c bzw. dessen Sockel 2c angeordnet, so daß sie einfach mit dem Fuß betätigt werden können. Selbstverständlich können die Schalter 21c, 22c an jeder anderen geeigneten Stelle des Schrankes 1c angeordnet sein. Die Mikroschalter 21c, 22c sind außerdem an eine (nicht dargestellte) Druckluftquelle angeschlossen. Auch die Pumpvorrichtungen 19c, 20c sind an die Druckluftquelle angeschlossen. Die Schalter 21c, 22c liegen, wie sich aus Figur 6 ergibt, hinter jeweils einer Sockelblende 41c und 42c, die schwenkbar am Sockel 2c gelagert sind. Wird die jeweilige Sockelblende 41c bzw. 42c geschwenkt, wird ein Stößel 43c, 44c des jeweiligen Schalters 21c, 22c durch die Sockelblende in den Mikroschalter verschoben und damit in bekannter Weise betätigt. Dadurch wird die zugehörige Pumpvorrichtung 19c, 20c eingeschaltet. Ihr druckseitiger Anschluß ragt in den Behälter 12'c, 13'c, so daß der Behälterinhalt unter Druck gesetzt wird. Er wird dadurch in die Steigleitung 19c, 20c gedrückt, in der er zum Auslaufstutzen 10c, 11c hochsteigt. Der Schalter 21c, 22c bzw. die Pumpvorrichtung 19c, 20c sind so ausgebildet, daß der Druck nur über eine kurze Zeit vorhanden ist. Dies hat den Vorteil, daß am Auslaufstutzen 10c, 11c das jeweilige hochgepumpte Mittel nur portionsweise austritt. Die Steuerung ist so vorgesehen, daß selbst bei ständiger Beaufschlagung des Schalters 21c, 22c die Pumpvorrichtung 19, 20 nur kurzzeitig eingeschaltet bleibt.

Neben den Schaltern 21c, 22c sind auf gleicher Höhe und mit Abstand voneinander und von den Schaltern 21c, 22c zwei weitere Schalter 25c und 26c vorgesehen. Der Schalter 26c ist über Druckluftleitungen 28'c, 28''c mit luftgesteuerten Ventilen 29c und 30c zweier Pumpen 31c und 32c zur Zuführung von Warmwasser und Kaltwasser zum Hahn 9c verbunden. Die Ventile 29c und 30c sind über Zuleitungen 33c und 34c an den Wasserhahn 9c angeschlossen. Der andere Schalter 25c ist mit einem Hubzylinder 36c über eine Druckluftleitung 27 verbunden.

Unmittelbar benachbart zur Seitenwand 4c ist im Unterschrank 1c ein Querträger 35c angeordnet, der etwa in halber Höhe des Schrankes liegt. An ihm ist der Hubzylinder 36c angelenkt, dessen Kolbenstange 36'c an einem nach hinten in Richtung auf die Rückwand 5 weisenden Arm 37c einer Klappe 38c angreift. Sie liegt in der Vorderfront des Unterschrankes 1c. Bei geöffneter Klappe (Figur 6) kann durch die gebildete Öffnung 39c z.B. schmutzige Wäsche oder andere Abfallprodukte in einen im Schrank vorgesehenen Eimer oder dgl. geworfen werden. Zum Öffnen der Klappe 38c muß nur über den Schalter 25c der Hubzylinder 36c betätigt werden. Der Schalter 25c läßt sich wie die übrigen Schalter 21c, 22c, und 26c mit dem Fuß einfach und schnell ein- und ausschalten. Die Schalter 25c, 26c liegen wie die anderen Schalter 21c, 22c im Schwenkbereich jeweils einer Sockelblende 45c und 46c, die im Ausführungsbeispiel zwischen den Sockelblenden 41c, 42c liegen. Werden sie geschwenkt, wird der jeweils hinter ihnen angeordnete luftgesteuerte Schalter 25c, 26c betätigt, der vorzugsweise ein Mikroschalter ist. Mit dem Schalter 26c werden die luftgesteuerten Ventile 29c, 30c umgeschaltet, so daß das Wasser über die Zuleitungen 33c, 34c zum Wasserhahn 9c strömt. Durch Betätigen des Schalters 25c wird der Pneumatikzylinder beaufschlagt, dessen Kolbenstange 36'c ausfährt und die Klappe 38c in die in Figur 6 dargestellte Öffnungsstellung schwenkt.

Die Klappe 38c kann sich über die ganze Breite, aber auch nur über einen Teil der Breite der Vorderwand 6c erstrecken und ist mit ihrem unteren Längsrand an der Vorderwand 6c angelenkt. Vorteilhaft weist die Klappe 38c an beiden Enden Seitenwände 47c (Figur 6) auf, die sich von der Klappe aus in Richtung auf den Schrank 1c erstrecken und bei geöffneter Klappe zusammen mit ihr einen Einfüllbereich begrenzen.

Unmittelbar unterhalb der Klappe 38c weist der Schrank 1c eine Türe 40c auf, über die sich der Abfall- bzw. Sammelbehälter, der über die Klappe 38c gefüllt werden kann, zum Entleeren aus dem Schrank herausholen läßt.

Da die Schalter und Ventile luftbetätigt werden, kann der Schrank dort eingesetzt werden, wo eine entsprechende Druckluftversorgung gewährleistet ist, beispielsweise in Zahnarztpraxen.

## Patentansprüche

1. Einrichtung, vorzugsweise schrankartiges Möbel für Arzt-/Zahnarztpraxen, mit mindestens einem zu betätigenden Teil (2, 2a, 2b; 12c, 13c; 38c), an dem mindestens eine druckluftgesteuerte Antriebsvorrichtung (3, 3a, 3b; 19c, 20c) angreift, die über einen Auslöser (4, 5, Z; 4a, 5a; 21c, 22c) betätigbar ist, der an ein Schaltventil (6, 6a) angeschlossen ist, das mit der Antriebsvorrichtung (3, 3a, 3b; 19c, 20c) verbunden ist,
dadurch gekennzeichnet, daß der Auslöser (4, 5, Z; 4a, 5a; 21c, 22c) ein Druckschalter ist, der bei einer ersten Betätigung einen ersten Schaltimpuls erzeugt, durch den die Antriebsvorrichtung (3, 3a, 3b; 19c, 20c) eine erste Antriebsfunktion ausführt, und daß der Auslöser bei einer zweiten Betätigung einen zweiten Schaltimpuls erzeugt, durch den die Antriebsvorrichtung (3, 3a, 3b; 19c, 20c) eine zweite Antriebsfunktion ausführt.

2. Einrichtung, an der über einen Beschlag das betätigbare Teil, insbesondere eine Schublade, angeordnet ist, nach Anspruch 1,
dadurch gekennzeichnet, daß die Antriebsvorrichtung (3, 3a, 3b) das betätigbare Teil (2, 2a, 2b) aus der Öffnungs- in die Schließstellung und umgekehrt verstellt.

3. Einrichtung nach Anspruch 1 oder 2,
dadurch gekennzeichnet, daß die Antriebsvorrichtung (3, 3a, 3b) eine pneumatisch arbeitende Kolben-Zylinder-Einheit ist.

4. Einrichtung nach Anspruch 3,
dadurch gekennzeichnet, daß sich die Kolben-Zylinder-Einheit in Verstellrichtung (P) des bewegbaren Teiles (2) erstreckt.

5. Einrichtung nach Anspruch 3,
dadurch gekennzeichnet, daß sich die Kolben-Zylinder-Einheit (3a, 3b) quer zur Verstellrichtung (P) des bewegbaren Teiles (2a, 2b) erstreckt.

6. Einrichtung nach einem der Ansprüche 3 bis 5,
dadurch gekennzeichnet, daß der Zylinder (7, 7a, 7b) der Kolben-Zylinder-Einheit (3, 3a, 3b) an einer Rückwand (9a, 9b) und/oder einer Seitenwand (11) der Einrichtung (1, 1a, 1b) angeordnet ist.

7. Einrichtung nach einem der Ansprüche 3 bis 6,
dadurch gekennzeichnet, daß die Kolbenstange (8, 8a) der Kolben-Zylinder-Einheit (3, 3a) am bewegbaren Teil (2, 2a) angreift.

8. Einrichtung nach einem der Ansprüche 3, 4, 6 oder 7, bei dem das bewegbare Teil eine Schublade und das Gestell ein Möbelkorpus ist, dadurch gekennzeichnet, daß die Kolben-Zylinder-Einheit (3) zwischen der Schublade (2) und einer Einrichtungsseitenwand (11) liegt, und daß die Kolbenstange (8) an einer in Verstellrichtung (P) vorderen Verbindungsstelle (10), vorzugsweise der Blende, der Schublade (2) befestigt ist.

9. Einrichtung nach einem der Ansprüche 3 und 5 bis 8, bei dem das bewegbare Teil eine Schublade und das Gestell ein Möbelkorpus ist,
dadurch gekennzeichnet, daß die Kolbenstange (8a) an der Rückwand (17) der Schublade (2a) angelenkt ist.

10. Einrichtung nach einem der Ansprüche 3 und 5 bis 7,
dadurch gekennzeichnet, daß die Kolben-Zylinder-Einheit (3b) senkrecht zur Verstellrichtung (P) des bewegbaren Teiles (2b) liegt.

11. Einrichtung nach Anspruch 10, dadurch gekennzeichnet, daß die Kolben-Zylinder-Einheit (3b) zwei synchron gegenläufig bewegbare Kolbenstangen (8b, 8b') hat, an deren Enden ein scherenartig ausgebildetes Kraftübertragungsteil (27) angelenkt ist, das vorzugsweise am bewegbaren Teil (2b) angelenkt ist.

12. Einrichtung nach Anspruch 10 oder 11,
bei dem das bewegbare Teil eine Schublade und das Gestell ein Möbelkorpus ist, dadurch gekennzeichnet, daß der Zylinder (8b) der Antriebsvorrichtung (3b) zwischen der Einrichtungsrückwand (9b) und einer Rückwand (17b) der Schublade (2b) angeordnet ist, und daß das Kraftübertragungsteil (27) an einer vorderen Blende (10b) der Schublade (2b) angelenkt ist.

13. Einrichtung nach einem der Ansprüche 10 bis 12,
dadurch gekennzeichnet, daß am verstellbaren Teil (2b) ein Zusatzantrieb (39) angreift, der vorzugsweise eine Kolben-Zylinder-Einheit ist.

14. Einrichtung nach Anspruch 13, dadurch gekennzeichnet, daß der Zusatzantrieb (39) in Verstellrichtung (P) des verstellbaren Teils (2b) liegt.

15. Einrichtung nach Anspruch 13 oder 14, dadurch gekennzeichnet, daß im Bewegungsweg der Kolbenstange (41) des Zusatzantriebes (39) das verstellbare Teil (2b) liegt.

16. Einrichtung nach einem der Ansprüche 13 bis 15,
dadurch gekennzeichnet, daß die Kolbenstange (41) am freien Ende einen Puffer (42) aufweist, der bei eingefahrener Schublade (2b), vorzugsweise an deren Rückwand (17b) anliegt.

17. Einrichtung nach einem der Ansprüche 1 bis 16,
dadurch gekennzeichnet, daß zur Einstellung der Ausfahrgeschwindigkeit der Kolbenstange (8, 8a, 8b, 8b') der Kolben-Zylinder-Einheit (3, 3a, 3b) mindestens ein Einstellteil, vorzugsweise eine Drossel, vorgesehen ist.

18. Einrichtung nach einem der Ansprüche 1 bis 17, dadurch gekennzeichnet, daß die Antriebsvorrichtung elektromotorisch betätigbar ist.

19. Einrichtung nach einem der Ansprüche 1 bis 18, dadurch gekennzeichnet, daß die Antriebsvorrichtung ein Reibrad aufweist, das auf einer Welle eines Elektromotors sitzt und mit dem bewegbaren Teil und/oder dem Gestell zusammenwirkt.

20. Einrichtung nach einem der Ansprüche 1 bis 19,
dadurch gekennzeichnet, daß der Auslöser (4, 5) im Bodenbereich und/oder in Kniehöhe der Einrichtung (1, 1a, 1b) angeordnet und vorzugsweise eine Schaltleiste ist.

21. Einrichtung nach einem der Ansprüche 1 bis 20,
dadurch gekennzeichnet, daß das zu betätigende Teil (12c, 13c) ein Spender ist, der zum Fördern des Spendermittels mit der als Pumpvorrichtung (19c, 20c) ausgebildeten Antriebsvorrichtung versehen ist, die druckluftgesteuert und an einem von außen betätigbaren Schalter (21c, 22c) als Auslöser angeschlossen ist, der ebenfalls druckluftgesteuert ist.

22. Einrichtung nach Anspruch 21, dadurch gekennzeichnet, daß der Auslauf (10c, 11c) mit einer in den Spender (12c, 13c) ragenden Zuführleitung verbunden ist, die vorzugsweise eine Steigleitung (14c, 15c) aufweist, die vorzugsweise von einer Fülleitung (16c, 17c) mit Abstand umgeben ist.

23. Einrichtung nach Anspruch 22, dadurch gekennzeichnet, daß sich die Fülleitung (16c, 17c) zwischen dem Behältnis (12a',13a') und dem Auslauf (10c, 11c) erstreckt.

24. Einrichtung nach einem der Ansprüche 21 bis 23,
dadurch gekennzeichnet, daß der Schalter (21c, 22c) ein Mikroschalter ist.

25. Einrichtung nach einem der Ansprüche 21 bis 24,
dadurch gekennzeichnet, daß ein zweiter, im wesentlichen gleich ausgebildeter Spender (12c, 13c) vorgesehen ist, der vorzugsweise im wesentlichen spiegelsymmetrisch zum anderen Spender (13c, 12c) angeordnet ist.

26. Einrichtung nach Anspruch 25, dadurch gekennzeichnet, daß zum Betätigen des weiteren Spenders (12c, 13c) ein zweiter druckluftgesteuerter Schalter (21c, 22c), vorzugsweise ein Mikroschalter, vorgesehen ist.

27. Einrichtung nach einem der Ansprüche 21 bis 26, dadurch gekennzeichnet, daß sie mindestens einen Wasserhahn (9c) aufweist, an den mindestens eine Zuleitung, vorzugsweise eine Kalt- und eine Warmwasserleitung (33c, 34c), angeschlossen ist, und daß in der Leitung (33c, 34c) ein vorzugsweise luftgesteuertes Ventil (29c, 30c) liegt, das vorzugsweise über einen druckluftgesteuerten Schalter (26c), vorzugsweise einen Mikroschalter, betätigbar ist.

28. Einrichtung nach einem der Ansprüche 25 bis 27,
dadurch gekennzeichnet, daß beide Spender (12c, 13c) im wesentlichen gleich ausgebildet sind.

29. Einrichtung nach einem der Ansprüche 21 bis 28,
dadurch gekennzeichnet, daß die Einrichtung (1c) eine Vorderwand (6c), eine Rückwand (5c) und eine Seitenwand (3c, 4c) hat.

30. Einrichtung nach Anspruch 29, dadurch gekennzeichnet, daß an der Vorderwand (6c) eine Klappe (38c) schwenkbar gelagert ist, an der vorzugsweise ein Hubzylinder (36c) angreift, der innerhalb der Einrichtung (1c), vorzugsweise auf einem Querträger (35c), angeordnet und vorzugsweise über einen Schalter (25c), vorzugsweise einen Mikroschalter, luftgesteuert betätigbar ist.

31. Einrichtung nach einem der Ansprüche 21 bis 30,
dadurch gekennzeichnet, daß mindestens ein, vorzugsweise alle Schalter (21c, 22c, 25c, 26c) an einem Sockel (2c) der Einrichtung (1c) vorgesehen sind, vorzugsweise jeweils hinter einer schwenkbaren Sockelblende (41c, 42c, 45c, 46c) liegen.

32. Einrichtung nach Anspruch 30 oder 31, dadurch gekennzeichnet, daß unterhalb der Klappe (38c) eine Tür (40c) angeordnet ist.

## Claims

1. Device, a cupboard-shaped cabinet preferably, for medical and dental surgeries with at least one part which can be operated (2, 2a, 2b; 12c, 13c; 38c) to which at least one pneumatically working driving arrangement (3, 3a, 3b; 19c, 20c) is connected which can be operated by a tripping device (4, 5, z; 4a, 5a; 21c, 22c) which is connected to the pilot valve (6, 6a) which is connected to the driving arrangement (3, 3a, 3b; 19c, 20c), characterized in that the tripping device (4, 5, z; 4a, 5a; 21c, 22c) is a pressure switch which produces, at first operation, a first switching pulse through which the driving arrangement produces a first driving function and that the tripping device produces, at second operation, a second switching pulse through which the driving arrangement (3, 3a, 3b; 19c, 20c) produces a second driving function.

2. Device to which, through a fixture, the part which is to be operated , especially a drawer, is fixed,according to claim 1, characterized in that the driving arrangement (3, 3a, 3b) moves the operating part from the open position into the closed position and vice-versa.

3. Device according to any one of claims 1 or 2, characterized in that the driving arrangement ( 3, 3a, 3b) is a pneumatically operated piston-cylinder-unit.

4. Device according to claim 3, characterized in that the piston-cylinder-unit extends into the direction (P) of the mobile part (2).

5. Device according to claim 3, characterized in that the piston-cylinder-unit extends transversely (P) to the direction of adjustment of the mobile part (2a, 2b).

6. Device according to any one of claims 3 to 5,characterized in that the cylinder (7, 7a, 7b) of the piston-cylinder-unit(3, 3a, 3b) is arranged to a back wall (9a,9b) and/or a side wall (11) of the device (1, 1a, 1b).

7. Device according to any one of claims 3 to 6, characterized in that the piston rod (8, 8a) of the piston-cylinder-unit (3, 3a) attacks the mobile part (2, 2a).

8. Device according to any one of claims 3, 4, 6 or 7, characterized in that the mobile part is a drawer and the frame is a housing characterized in that the piston-cylinder-unit (3) is based between the drawer (2) and one of the device's walls (11) and the piston rod (8) is fixed to a font joint which is based at the direction of adjustment (10) which is preferably the panel of the drawer.

9. Device according to one of claims 3 and 5 to 8, characterized in that the mobile part is a drawer and the frame is a housing, characterized in that the piston rod is fixed to the back wall of the drawer.

10. Device according to any one of claims 3 and 5 to 7, characterized in that the piston-cylinder-unit (3b) is square with the direction of adjustment (P) of the moving part (2b).

11. Device according to claim 10, characterized in that the piston-cylinder-unit (3b) has 2 synchronously moving piston rods, working in opposite direction (8a, 8b). At their ends, a power transmission device (27) which is scissor-shaped is fixed which is, preferably, fixed to the mobile part (2b).

12. Device according to any one of claims 10 or 11, characterized in that the mobile part is a drawer and the frame is a housing, characterized in that the cylinder (8b) of the driving arrangement (3b) is situated between the device's back wall (9b) and one back wall (17b) of the drawer (2b). The power transmission device (27) is fixed to a front panel (10b) of the drawer.

13. Device according to any one of claims 10 to 12, characterized in that the adjustable part (2b) is attached to an additional drive (39) which is, preferably, a piston-cylinder-unit.

14. Device according to claim 13, characterized in that the additional drive is based in the direction of adjustment of the adjustable part (2b).

15. Device according to any one of claims 13 or 14, characterized in that within the piston-rod's way to move of the additional drive (39) the adjustable part (2b) is situated.

16. Device according to any one of claims 13 to 15, characterized in that the piston rod (41) has a pad (42) on its end which, if the drawer is closed (2b), should be fixed, preferably, at its back side (17).

17. Device according to any one of claims 1 to 16, characterized in that that in order to regulate the out-going speed of the carriage of the piston rod of the piston-cylinder-unit, (8, 8a, 8b, 8b'), at least one adjusting device, preferably a choke, is provided.

18. Device according to any one of claims 1 to 17, characterized in that the driving arrangement can be operated electromotively.

19. Device according to amy one of claims 1 to 18, characterized in that the driving arrangement contains a friction wheel which is placed on a shaft of an electic motor and interacts with the mobile part and/or the frame.

20. Device according to any one of claims 1 to 19, characterized in that the release knob (4,5) is situated in the region of the floor and/or is situated near the knee-hight of the device (1, 1a, 1b) and is, preferably, a media channel.

21. Device according to any one of claims 1 to 20, characterized in that the part to be operated (12c, 13c) is a dispenser. In order to dispense the liquid, it is provided with a pneumatically working driving arrangement formed as a pumping device (19c, 20c) connected to an outer switch (21c, 22c) which is the tripping device which is also operated pneumatically.

22. Device according to claim 21, characterized in that the out flow (10c, 11c) is connected to a supply lead which leads into the dispenser (12c, 13c), which is a stand-pipe, (14c, 15c) which is, preferably surrounded, at a distance, by a filling pipe line (16c, 17c).

23. Device according to claim 22, characterized in that the filling pipe line (16c, 17c) is situated between the box (12a', 13a') and the out flow. (10c, 11c)

24. Device according any one of claims 21 to 23, characterized in that the switch (21c, 22c) is a mirco-switch.

25. Device according to any one of claims 21 to 24, characterized in that a second dispenser which is basically identical to the first one is provided (13c, 12c). Preferably, it is situated symmetrically to the other one.

26. Device according to claim 25 characterized as follows: In order to use the second distributor (12c, 13c), which is operated pneumatically by a switch (21c, 22c) a second switch, which is preferably a micro one, is provided.

27. Device according to any one of claims 21 to 26, characterized in that the device has to have at least one water tap(9c) to which at least one delivery pipe , preferably one for cold water and one for warm water is connected.(33c,34c). Within the pipe line, (33c,34c) there has to be, preferably, an air-controlled valve (29c, 30c) which is, preferably, operated by a pneumatic switch (26c),which is, preferably, a micro switch.

28. Device according to any one of claims 25 to 27, characterized in that both dispensers (12c, 13c) are basically identical.

29. Device according to any one of claims 21 to 28, characterized in that the device (1c) consists of a front wall (6c), a back wall (5c) and a side wall (3c,4c).

30. Device according to claim 29, characterized in that at the front wall (6c), there is a swingable flap (38c) which is, prefeably attacked by a lifting cylinder (36c) which is situated within the device, preferably on a corssbar (35c) and is operated by an air-controlled switch (25c) which is, preferably a micro switch.

31. Device according to any one of claims 21 to 30, characterized in that at least one, preferably all switches (21c, 22c,25c, 26c) are provided to be situated at the base (2c) of the device (1c), preferably each behind a swingable base panel (41c, 42c,45c, 46c).

32. Device according to any one of claims 30 or 31, characterized in that below the flap (38c), a door (40c) is situated.

## Revendications

1. Dispostitf, de préférence un élément de meuble, destiné aux cabinets médicaux et dentaires avec au moins une partie à actionner ( 2, 2a, 2b; 12c, 13 c; 38 c) à laquelle un méchanisme de commande pneumatique ( 3, 3a, 3b; 19c, 20 c) est appliqué qui peut être actionné par un déclencheur(4, 5, Z; 4a, 5a;21 c, 22c) qui est raccordé à un relais capteur qui est lié au méchanisme de commande; est caractérisé en ce que le déclencheur (4,5,z; 4a, 5a; 21c, 22c) est un interrupteur à pression qui exerce, à la première mise en action une première impulsion par laquelle le méchanisme de commande ( 3, 3a, 3b; 19c, 20c) exerce sa première fonction de commande et que le déclencheur exerse à la deuxième mise en action une deuxième impulstion par laquelle le méchanisme de commande (3, 3a, 3b; 19c, 20c) exerce une deuxième fonction de commande.

2. Dispositif auquel la partie à actionner, surtout un tiroir, est disposé par une garniture selon revendication 1 est caractérisé en ce que le méchanisme de commande (3, 3a, 3b) règle la partie à actionner (2, 2a, 2b) de l'ouverture à la fermeture du tiroir et inversement.

3. Dispositif selon la revendication 1 ou 2 caractérisé en ce que le méchanisme de commande ( 3, 3a, 3b) est une unité piston-cylindre pneumatique.

4. Dispositif selon la revendication 3 caractérisé en ce que l'unité piston-cylindre est disposée en direction de réglage (P) de la partie mobile.

5. Dispositif selon la revendication 1, caractérisé en ce que l'unité piston-cylindre (3a, 3b) est disposée en direction transversale par rapport à la direction de réglage (P) de la partie mobile (2a, 2b).

6. Dispositif selon une des revendications 3 à 5, caractérisé en ce que le cylindre (7, 7a, 7b) de l'unité piston-cylindre (3, 3a, 3b) est disposé sur une face arrière (9a, 9b) et/ou sur une paroi latérale (11) de l'équipement (1, 1a, 1b).

7. Dispositif selon une des revendications 3 à 6, caractérisé en ce que la tige de piston (8, 8a) de l'unité piston-cylindre (3, 3a) est appliquée à la partie mobile (2, 2a).

8. Dispositif selon une des revendications 3, 4, 6 ou 7 dont la partie mobile est représentée par un tiroir et le support par un corps de meuble. Le dispositif présente les caractéristiques suivantes: L'unité piston-cylindre (3) est positionnée entre le tiroir (2) et une paroi latérale (11) du meuble et la tige de piston (8) est fixée à une pièce de jonction (10), de préférence à un panneau, se trouvant en amont du tiroir (2) en direction du réglage (P).

9. Dispositif selon une des revendications 3 et 5 à 8, que la partie mobile est représentée par un tiroir et le support par un corps de meuble, est caractérisé en ce que le tige de piston (8a) est appliqué à la face arrière (17) du tiroir (2a).

10. Dispositif selon une des revendications 3 et 5 à 7, caractérisé en ce que l'unité piston-cylindre (3b) est positionnée en ligne verticale par rapport à la direction de réglage (P) de la partie mobile (2b).

11. Dispositif selon la revendication 10, caractérisé en ce que l'unité piston-cylindre (3b) a deux tiges de piston mobiles (8b, 8b') à fonctionnement synchrone opposé aux bouts desquelles est articulée une pièce de transmission (27) en forme de ciseaux articulée de préférence à la partie mobile (2b).

12. Dispositif selon les revendications 10 ou 11, caractérisé en ce que la partie mobile est représentée par un tiroir et le support par un corps de meuble.
Le dispositif présente les caractéristiques suivantes: Le cylindre (8b) du mécanisme de commande (3b) est disposé entre la face arrière (9b) du meuble et une face arrière (17b) du tiroir (2b) et la pièce de transmission (27) est articulée à un panneau de front (10b) du tiroir (2b).

13. Dispositif selon une des revendications 10 à 12, caractérisé en ce qu' à la partie mobile (2b) est appliquée un mécanisme de commande supplémentaire (39), de préférence une unité piston-cylindre.

14. Dispositif selon la revendication 13, caractérisé en ce que le mécanisme de commande supplémentaire (39) est placé dans la direction (P) de la partie mobile (2b).

15. Dispositif les revendications 13 ou 14, caractérisé en ce que la partie mobile (2b) se trouve dans le parcours de mouvement de la tige de piston (41) du mécanisme de commande (39).

16. Dispositif selon une des revendications 13 à 15, caractérisé en ce que la tige de piston (41) est pourvue,à son bout libre, d'un butoir (42) adhérent de préférence à la face arrière (17b) du tiroir (2b) en position rentrée.

17. Dispositif selon une des revendications 1 à 16, caractérisé en ce que pour régler la vitesse de sortie de la tige de piston (8, 8a, 8b, 8b') de l'unité piston-cylindre (3, 3a, 3b), il est prévu au minimum une pièce de réglage, de préférence un dispositif d'étranglement.

18. Dispositif selon une des revendications 1 à 17, caractérisé en ce que le mécanisme de commande est réglable par un moteur électrique.

19. Dispositif selon une des revendications 1 à 18, caractérisé en ce que le mécanisme de commande est pourvu d'une roue abrasive montée à une arbre d'un moteur électrique et concourant avec la partie mobile et/ou le support.

20. Dispositif selon une des revendications 1 à 19, caractérisé en ce que le déclencheur (4, 5) est disposé près du sol et/ou à la hauteur du genou du meuble (1, 1a, 1b), de préférence sous forme d'un listel de réglage.

21. Dispositif selon une des revendications 1 à 20, caractérisé en ce que la partie à actionner (12c, 13c) est un distributeur qui est equippé, pour distribuer du produit, avec un méchanisme de commande sous forme d'une installation de pompage pneumatique (19c, 20c) et qui est raccordée à un interrupteur extérieur (21c,22c) qui sert comme déclencheur qui est également pneumatique.

22. Dispositif selon la revendication 21, caractérisé en ce que la sortie (10c, 11c) est raccordée à une conduite d'alimentation passant dans le distributeur (12c, 13c) qui dispose de préférence d'un tuyau ascendant (14, 15c) entouré, à distance,de préférence d'un tuyau de remplissage (16c, 17c).

23. Dispositif la revendication 22, caractérise en ce que le tuyau de remplissage (16c, 17c) est situé entre le récipient (12a', 13a') et la sortie (10c, 11c).

24. Dispositif selon une des revendications 21 à 23, caractérisé en ce que le commutateur (21c, 22c) est un microrupteur.

25. Dispositif selon une des revendications 21 à 24, caractérisé en ce qu' il est prévu un deuxième distributeur(12c, 13c) du même genre qui est disposé de préférence symétriquement par rapport au premier distributeur (13c, 12c).

26. Dispositif selon la revendication 25,caractérisé en ce que pour actionner le deuxième distributeur (12c, 13c), il est prévu un deuxième commutateur (21c, 22c) pneumatique, de préférence un microrupteur.

27. Dispositif selon une des revendications 21 à 26,caractérisé en ce qu'il y a au moins un robinet (9c) auquel est raccordée au moins une conduite d'alimentation, de préférence une conduite d'eau froide et d'eau chaude (33c, 34c). A l'intérieur de la conduite (33c, 34c) se trouve une soupape de préférence pneumatique (29c, 30c) qui est actionnée de préférence par un commutateur pneumatique (26c), de préférence un microrupteur.

28. Dispositif selon une des revendications 25 à 27, caractérisé en ce que les deux distributeurs (12c, 13c) sont du même genre.

29. Dispositif selon une des revendications 21 à 28, caractérisé en ce que le dispositif (1c) a une face avant (6c), une face arrière (5c) et une face latérale (3c, 4c).

30. Dispositif selon le revendication 29 caractérisé en ce qu'à la face avant (6c) se trouve un clapet pivotant (38c) commandé de préférence par un vérin hydraulique (36c) placé à l'intérieur du dispositif (1c), de préférence sur une traverse (35c) et qui peut être actionné de préférence par un commutateur pneumatique(25c), de préférence par un microrupteur.

31. Dispositif selon une des revendications 21 à 30, caractérisé en ce qu'au moins un commutateur, de préférence tous les commutateurs (21c, 22c, 25c, 26c) sont disposés au socle (2c) du dispositif (1c), de préférence derrière un panneau amovible (41c, 42c, 45c, 46c).

32. Dispositif selon une des revendications 30 ou 31,caractérisé en ce qu' au-dessous du clapet (38c) est disposé une porte (40c).
